Europäisches Patentamt

European Patent Office

Office européen des brevets

(11) Publication number: **0 312 249**
**A1**

## (12) EUROPEAN PATENT APPLICATION

(21) Application number: **88309288.4**

(22) Date of filing: **06.10.88**

(51) Int. Cl.⁴: **A61K 33/08** , //(A61K33/08,
**31:70,31:19,31:045)**

(30) Priority: **14.10.87 JP 260726/87**

(43) Date of publication of application:
**19.04.89 Bulletin 89/16**

(84) Designated Contracting States:
**AT BE CH DE FR GB IT LI LU NL SE**

(71) Applicant: **Takeda Chemical Industries, Ltd.**
**27, Doshomachi 2-chome Higashi-ku**
**Osaka-shi Osaka, 541(JP)**

(72) Inventor: **Nonomura, Muneo**
**4-204, 6 Satsukigaoka-nishi**
**Suita Osaka 565(JP)**
Inventor: **Yamada, Masayuki**
**11-6, Daiwanishi 2-chome**
**Kawanishi Hyogo 666-01(JP)**

(74) Representative: **Lewin, John Harvey et al**
**ELKINGTON AND FIFE Beacon House 113**
**Kingsway**
**London WC2B 6PP(GB)**

(54) **Aqueous pharmaceutical preparation for oral administration.**

(57) The aqueous pharmaceutical preparation for oral administration containing calcium ion and magnesium ion in high concentration according to this invention has had the bitter or repugnant taste of the metal ions effectively masked, does not undergo precipitation, and can be safely administered with ease.

EP 0 312 249 A1

EP 0 312 249 A1

## Aqueous Pharmaceutical Preparation for Oral Administration

This invention relates to an aqueous pharmaceutical preparation for oral administration which comprises calcium ion and magnesium ion in high concentrations.

Recently much light has been shed on the relationship between a low intake of calcium and magnesium ions with a high incidence of cardiovascular disorders and the importance of a simultaneous intake of calcium and magnesium ions, not an intake of calcium ion alone, has been pointed out.

By way of illustration, it has been reported that the lower the Ca/Mg intake ratio, the higher was the mortality due to ischemic heart diseases [Advances in Cardiology, 25, 9-18 (1978)] and that the myocardial Mg concentration was low in patients succumbing to death owing to cardiopathy [National Council of Canada NRCC 17581, 1979].

It is also considered that in the prevention and treatment of osteoporosis as well as in calcium supplementation in pregnant women, the administration of calcium and magnesium in an appropriate ratio will contribute to a more effective intake of these metal ions.

For these purposes, it seems more desirable, from the standpoints of the ease of administration and the high absorption rate, to administer an aqueous preparation containing high concentrations of calcium and magnesium ions by the oral route. At present, however, no such preparation is available.

There is the literature describing an infusion [Japanese Unexamined Patent Publication No. 30522/1986] or aqueous injection [PTC Publication WO82/03552] for caloric supplementation which contains calcium and magnesium ions as electrolytes in addition to carbohydrate but the concentrations of metal ions in these preparations are absolutely low and in terms of taste, too, such compositions are not suited for oral administration of calcium and magnesium ions.

There is also known an aqueous electrolyte solution containing various metal ions and a glucose polymer [British Patent No;. 1506327] but here the concentrations of calcium and magnesium ions that can be incorporated are so low that the aforementioned object cannot be accomplished.

The present inventors took interest in the prevention and treatment of various diseases by simultaneous administration of calcium and magnesium ions in high concentrations and conducted a full-breadth investigation for the purpose of supplying the market with an aqueous preparation of these ions for oral administration.

In any aqueous composition containing high concentrations of calcium and magnesium ions, sedimentation and precipitation are liable to take place to detract from the marketability of the product and from the feeling of ingestion and the process of absorption of the ingredients.

Particularly when allowed to stand at a low temperature of -5°C to -10°C in the course of distribution and storage, such aqueous composition is readily frozen and the sediments and gels formed on thawing remain undissolved for many hours.

Furthermore, any aqueous preparation containing high concentrations of calcium and magnesium ions has an intense bitter, astringent or otherwise repugnant taste associated with these metals which make it either impossible or difficult to ingest orally.

To solve these problems, the present inventors conducted a thorough exploration in regard to the substances and quantities to be formulated and the method of formulation. This invention is the culmination of these research endeavors.

This invention provides an aqueous pharmaceutical composition for oral administration comprising

(i) a water-soluble polyhydric alcohol containing a maximum of 12 carbon atoms;

(ii) at least one organic acid selected from the group consisting of lactic acid, gluconic acid and ascorbic acid;

(iii) at least 0.4 w/v % of calcium ion; and

(iv) at least 0.1 w/v % of magnesium ion.

The aforesaid water-soluble polyhydric alcohol containing a maximum of 12 carbon atoms include, among others, non-reducing monosaccharides such as sorbitol, xylitol, mannitol, etc., reducing monosaccharides such as fructose, sorbose, ribulose, ribose, glucose, galactose, mannose, etc., disaccharides such as maltose, lactose, sucrose, maltitol, lactitol, etc., and polyols such as glycerin, propylene glycol and so on. These water-soluble polyhydric alcohols containing a maximum of 12 carbon atoms can be used alone or in combination.

These water-soluble polyhydric alcohols are used in a total proportion of generally 1 to 50 w/v % and preferably about 10 to 30 w/v %.

2

The aforesaid organic acid is one or more members selected from the group consisting of lactic acid, gluconic acid and ascorbic acid. From the standpoint of stability, solubility and taste, however, lactic acid is the most desirable. These organic acids are incorporated in a total proportion of generally 0.5 to 10 w/v % and preferably 1 to 5 w/v %.

The calcium ion source is added generally in the form of a salt and preferably is an organic acid salt of calcium such as calcium lactate, calcium gluconate and so on or an inorganic acid salt such as calcium chloride. The aforesaid calcium ion concentration of at least 0.4 w/v % is equivalent to at least 2.2 w/v % of calcium lactate. The preferred concentration of calcium lactate is 2.5 to 8 w/v % (0.45-1.45 w/v % as calcium ion).

The magnesium ion source is added generally in the form of an oxide, hydroxide, or organic or inorganic salt, such as magnesium oxide, magnesium hydroxide, magnesium aspartate, magnesium chloride and so on. Taking magnesium oxide as an example, the aforesaid magnesium ion concentration of at least 0.1 w/v % is equivalent to at least 0.15 w/v % of magnesium oxide. The preferred range is 0.15 to 0.6 w/v % as magnesium oxide (0.1 to 0.25 w/v % as magnesium ion).

The aforesaid calcium and magnesium ion sources can respectively be used alone or as a mixture of 2 or more different species within the aforementioned concentration range.

The preferred ratio of calcium ion to magnesium ion is Ca/Mg = about 3/1 to 1/1 (w/w).

In the aqueous pharmaceutical composition of this invention, there may optionally be incorporated edible water-soluble high molecular substances such as polyethylene glycol, polyvinyl alcohol, polyvinylpyrrolidone, soluble starch, methylcellulose, dextrin, purulan, pectin and so on. From the standpoint of convenience in use, polyethylene glycol, polyvinyl alcohol and polyvinylpyrrolidone are advantageous.

From the standpoint of prevention of precipitation, it is recommended that a water-soluble high molecular compound be added when any other polyhydric alcohol than non-reducing monosaccharide is incorporated.

As water-soluble high molecular compounds for this purpose, polyethylene glycol, polyvinylpyrrolidone and polyvinyl alcohol are particularly preferred.

These water-soluble high molecular compounds are added generally at the level of 0.05 to 5 w/v % and preferably at 0.1 to 3%.

It is also possible to further incorporate various preservatives such as benzoic acid and p-hydroxybenzoic acid, inclusive of their salts, p-hydroxybenzoic esters (methylparaben, propylparaben, butylparaben, etc.), various corrigents inclusive of artificial sweeteners such as saccharin, aspartame, etc. and natural sweeteners such as steviosides etc., and various flavorants inclusive of amino acids such as glycine, aspartic acid, glutamic acid, etc., inclusive of their salts, nucleotides such as Ribotide, etc. in appropriate proportions.

If desired, various perfumes, colorants such as riboflavine compounds, caramel, etc., and viscosity builders or rheology modifiers such as alginic esters, carboxymethylcellulose, etc. may be further incorporated.

The aqueous pharmaceutical composition of this invention is preferably adjusted to pH about 3 to 5. Though the addition of the aforesaid components brings the pH of the composition into the above range, the pH of the composition may be adjusted, if necessary, by adding a basic or an acid substance.

The aqueous pharmaceutical composition of this invention can be manufactured, for example, by the following process.

To hot purified water held at a temperature of at least 80°C is added the magnesium ion source and after stirring to disperse the latter, an organic acid is added to dissolve the magnesium ion source. Then, the calcium ion source and the preservative are added and dissolved. This aqueous solution is cooled to about 40°C and the water-soluble polyhydric alcohol, colorant, perfume, etc. are added and dissolved with stirring. Then, purified water is added to make the specified quantity at room temperature. If required, this aqueous composition is filtered through a membrane filter and distributed into glass bottles or plastic containers.

The thus-obtained aqueous pharmaceutical composition of this invention which contains high concentrations of calcium and magnesium ions has had the bitter, astringent, and otherwise repugnant taste associated with the metals effectively masked and has a refreshing taste, thus enabling an easy and effective oral intake of calcium and magnesium ions in a suitable ratio.

Furthermore, the composition does not undergo precipitation or gelation on freezing and thawing, so that the product may claim a high market acceptance and present no problem with administration.

The usual dose of this aqueous pharmaceutical composition for oral administration is 20 to 100 ml per day per adult.

Examples

The following examples are further illustrative of the invention.

## Example 1

| Calcium lactate | | 2.1 g |
|---|---|---|
| Magnesium oxide | | 0.17 g |
| Lactic acid | | 1.40 g |
| Sorbitol | | 7.0 g |
| Sucrose | | 3.0 g |
| Polyethylene glycol 6000 | | 0.1 g |
| Riboflavine | | 0.2 mg |
| Perfume | q.s. | |
| Benzoic acid | 30 mg | |
| Butylparaben | | 5.0 mg |
| Purified water to make | | 50 ml |

In 25 ml of purified water held at 90°C was dispersed magnesium oxide with stirring and lactic acid was then added to dissolve the magnesium oxide. Then, calcium lactate, benzoic acid and butylparaben were added and dissolved. After cooling to 40°C, sucrose, riboflavine and perfume were added and dissolved with stirring. After the mixture was cooled to room temperature, polyethylene glycol 6000 was added. Finally, purified water was added to make a total of 50 ml.

The above solution was filtered through a membrane filter and the filtrate was filled into a 70 ml glass bottle.

The above aqueous pharmaceutical composition was allowed to freeze at -15°C. When this frozen product was allowed to stand at room temperature for 1 hour, it re-assumed the original state of a clear aqueous solution.

## Example 2

| Calcium lactate | | 2.1 g |
|---|---|---|
| Magnesium oxide | | 0.17 g |
| Lactic acid | | 1.40 g |
| Sorbitol | | 7.0 g |
| Sucrose | | 3.0 g |
| Riboflavine | | 0.2 mg |
| Benzoic acid | 30 mg | |
| Butylparaben | | 5.0 mg |
| Purified water to make | 50 ml | |

## Example 3

| Calcium lactate | 2.3 g |
|---|---|
| Magnesium oxide | 0.17 g |
| Lactic acid | 1.40 g |
| Sucrose | 7.0 g |
| Polyethylene glycol 6000 | 0.5 g |
| Riboflavine | q.s. |
| Perfume | q.s. |
| Benzoic acid | 30 mg |
| Butylparaben | 5.0 mg |
| Purified water to make | 50 ml |

Example 4

| Calcium chloride (5 $H_2O$) | 0.1 g |
|---|---|
| Calcium lactate | 1.0 g |
| Magnesium L-aspartate | 0.05 g |
| Magnesium oxide | 0.15 g |
| Lactic acid | 1.2 g |
| Ascorbic acid | 0.5 g |
| Fluctose | 12 g |
| Polyvinyl alcohol | 0.05 g |
| Benzoic acid | 30 mg |
| Propylparaben | 5.0 mg |
| Mentha water (Japanese Pharmacopeia) | q.s. |
| Purified water to make | 50 ml |

Example 5

| Calcium lactate | 2.3 g |
|---|---|
| Calcium gluconate | 0.8 g |
| Magnesium chloride | 0.1 g |
| Magnesium oxide | 0.08 g |
| Lactic acid | 0.5 g |
| Ascorbic acid | 1.0 g |
| Glycerin | 3 g |
| Sucrose | 8 g |
| Polyethylene glycol 6000 | 0.6 g |
| Ethylparaben | 3.0 mg |
| Butylparaben | 3.5 mg |
| Benzoic acid | 10 mg |
| Riboflavin phosphate | q.s. |
| Purified water to make | 50 ml |

Example 6

| Calcium lactate | 1.1 g |
|---|---|
| Magnesium oxide | 0.07 g |
| Lactic acid | 0.5 g |
| Sucrose | 5 g |
| Propylene glycol | 5 g |
| Polyethylene glycol 1000 | 0.5 g |
| Ethylparaben | 0.3 mg |
| Butylparaben | 3.0 mg |
| Purified water to make | 50 ml |

Example 7

| Calcium lactate | 2.3 g |
|---|---|
| Magnesium oxide | 0.17 g |
| Lactic acid | 1.27 g |
| Sorbitol | 11.0 g |
| Polyethylene glycol 6000 | 0.4 g |
| Riboflavine | 0.2 mg |
| Benzoic acid | 30 mg |
| Propylparaben | 2.5 mg |
| Drink taste flavor A 22513 | 0.05 ml |
| Citras mix flavor A 22661 | 0.05 ml |
| Purified water to make | 50 ml |

Example 8

| Calcium lactate | 2.3 g |
|---|---|
| Magnesium oxide | 0.17 g |
| Lactic acid | 1.27 g |
| Sorbitol | 7.0 g |
| Sucrose | 3.0 g |
| Polyethylene glycol 6000 | 0.4 g |
| Riboflavine | 0.2 mg |
| Benzoic acid | 30 mg |
| Propylparaben | 2.5 mg |
| Drink taste flavor A 22513 | 0.05 ml |
| Citras mix flavor A 22661 | 0.05 ml |
| Purified water to make | 50 ml |

Comparative Example 1

6

EP 0 312 249 A1

| Calcium lactate | 1.8 g |
|---|---|
| Magnesium oxide | 0.15 g |
| Lactic acid | 1.3 g |
| Polyethylene glycol 4000 | 1.0 g |
| Aspartame | 5 mg |
| Benzoic acid | 30 mg |
| Propylparaben | 5 mg |
| Purified water to make | 50 ml |

Comparative Example 2

| Calcium lactate | 2.3 g |
|---|---|
| Magnesium chloride | 0.8 g |
| Sucrose | 15 g |
| Polyvinyl alcohol | 0.05 g |
| Benzoic acid | 30 mg |
| Butylparaben | 5 mg |
| Purified water to make | 50 ml |

The aqueous compositions manufactured according to Comparative Examples 1 and 2 yielded precipitates and gels on reconstitution from the frozen state and these precipitates and gels either failed to disappear or persisted for many hours. Furthermore, when the thawing temperature was low (0°C to 10°C), the precipitates and gels were not liquidated but rather increased in amounts.


## Claims

1. An aqueous pharmaceutical composition for oral administration comprising
   (i) a water-soluble polyhydric alcohol containing a maximum of 12 carbon atoms;
   (ii) at least one organic acid selected from the group consisting of lactic acid, gluconic acid and ascorbic acid;
   (iii) at least 0.4 w/v % of calcium ion; and
   (iv) at least 0.1 w/v % of magnesium ion.

2. The composition according to claim 1, wherein the polyhydric alcohol is non-reducing monosaccharide, reducing monosaccharide, disaccharide or polyol.

3. The composition according to claim 2, wherein the non-reducing monosaccharide is a member selected from the group consisting of sorbitol, xylitol and mannitol.

4. The composition according to claim 2, wherein the reducing monosaccharide is a member selected from the group consisting of fructose, sorbose, ribulose, ribose, glucose, galactose and mannose.

5. The composition according to claim 2, wherein the disaccharide is a member selected from the group consisting of maltose, lactose, sucrose, maltitol and lactitol.

6. The composition according to claim 2, wherein the polyol is glycerin or propylene glycol.

7. The composition according to claim 2, wherein the polyhydric alcohol is in combination of non-reducing monosaccharide with disaccharide, or of disaccharide with polyol.

8. The composition according to claim 1, which contains polyhydric alcohol in an amount of about 1 to 50 w/v %.

9. The composition according to claim 1, which contains organic acid in an amount of about 0.5 to 10 w/v %.

10. The composition according to claim 1, wherein a source of the calcium ion is added in a form of organic acid salt of calcium.

7

11. The composition according to claim 1, wherein a source of the magnesium ion is added in a form of oxide, hydroxide, organic salt or inorganic salt of magnesium.

12. The composition according to claim 1, wherein the ratio of calcium ion to magnesium ion is about 3:1 to 1:1 (w/w).

13. The composition according to claim 1, which further contains edible water-soluble high molecular substance.

14. The composition according to claim 1, which is adjusted to pH about 3 to 5.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | FR-A-2 364 657 (LEWIS/HOWE CO.) <br> * Page 3, line 9 - page 4, line 16; page 6, lines 13-23; page 46, lines 1-33 * <br> --- | 1-14 | A 61 K 33/08 // (A 61 K 33/08 A 61 K 31:70 A 61 K 31:19 A 61 K 31:045) |
| Y | US-A-3 579 634 (GARLAND RICHARD BROWN) <br> * Column 6, line 64 - column 7, line 6; column 9, line 50 - column 10, line 19; column 18, line 69 - column 19, line 34 * <br> ----- | 1-14 | |

TECHNICAL FIELDS
SEARCHED (Int. Cl.4)

A 61 K

The present search report has been drawn up for all claims

| Place of search | Date of completion of the search | Examiner |
|---|---|---|
| THE HAGUE | 23-01-1989 | BRINKMANN C. |